(19) Europäisches Patentamt
European Patent Office
Office européen des brevets

(11) EP 4 144 353 A1

(12) # EUROPEAN PATENT APPLICATION
published in accordance with Art. 153(4) EPC

(43) Date of publication:
**08.03.2023 Bulletin 2023/10**

(21) Application number: **21795973.3**

(22) Date of filing: **27.04.2021**

(51) International Patent Classification (IPC):
***A61K 31/4965*** (2006.01) ***A61K 31/07*** (2006.01)
***A61K 31/11*** (2006.01) ***A61K 31/121*** (2006.01)
***A61K 31/122*** (2006.01) ***A61K 31/137*** (2006.01)
***A61K 31/138*** (2006.01) ***A61K 31/216*** (2006.01)
***A61K 31/343*** (2006.01) ***A61K 31/351*** (2006.01)
***A61K 31/352*** (2006.01) ***A61K 31/357*** (2006.01)
***A61K 31/366*** (2006.01) ***A61K 31/395*** (2006.01)
***A61K 31/403*** (2006.01) ***A61K 31/4045*** (2006.01)
***A61K 31/4178*** (2006.01) ***A61K 31/4184*** (2006.01)
***A61K 31/4245*** (2006.01) ***A61K 31/426*** (2006.01)
***A61K 31/427*** (2006.01)

(52) Cooperative Patent Classification (CPC):
**A61K 31/07; A61K 31/11; A61K 31/121; A61K 31/122; A61K 31/137; A61K 31/138; A61K 31/216; A61K 31/343; A61K 31/351; A61K 31/352; A61K 31/357; A61K 31/366; A61K 31/395; A61K 31/403; A61K 31/4045;**

(Cont.)

(86) International application number:
**PCT/JP2021/016737**

(87) International publication number:
**WO 2021/221043 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2020 JP 2020080731**

(71) Applicant: **FUJIFILM Toyama Chemical Co., Ltd.**
**Chuo-ku**
**Tokyo 104-0031 (JP)**

(72) Inventors:
- **KOMENO Takashi**
  **Toyama-shi, Toyama 930-8508 (JP)**
- **YAMASHITA Nozomi**
  **Toyama-shi, Toyama 930-8508 (JP)**

(74) Representative: **Hartz, Nikolai**
**Wächtershäuser & Hartz**
**Patentanwaltspartnerschaft mbB**
**Weinstrasse 8**
**80333 München (DE)**

(54) # CORONAVIRUS INFECTION THERAPEUTIC AGENT FORMED THROUGH COMBINATION OF PYRAZINE DERIVATIVE AND ANOTHER CORONAVIRUS INFECTION THERAPEUTIC DRUG

(57) A therapeutic agent for coronavirus infection comprising a combination of a pyrazine derivative or a salt thereof and another therapeutic agent for coronavirus infection.

EP 4 144 353 A1

(52) Cooperative Patent Classification (CPC): (Cont.)
**A61K 31/4178; A61K 31/4184; A61K 31/4245;
A61K 31/426; A61K 31/427; A61K 31/436;
A61K 31/439; A61K 31/4706; A61K 31/4965;
A61K 31/506; A61K 31/517; A61K 31/519;
A61K 31/522; A61K 31/5365; A61K 31/5377;
A61K 31/56; A61K 31/573; A61K 31/58;
A61K 31/59; A61K 31/661; A61K 31/662;
A61K 31/7056; A61K 31/706; A61K 31/716;
A61K 36/04; A61K 36/19; A61K 38/06;
A61K 38/12; A61K 38/17; A61K 38/21;
A61K 45/00; A61P 31/14; A61P 43/00**

## Description

### Technical Field

The present invention relates to a therapeutic agent for coronavirus infection comprising a combination of a pyrazine derivative or a salt thereof and one or more other therapeutic agents for coronavirus infection.

### Background Art

The novel coronavirus (SARS-CoV-2) first reported in China at the end of 2019 (Non-patent Literature 1) is an RNA virus belongs to the family *coronaviridae,* order *nidovirales* (Non-patent Literature 2). When infected with this virus, fever and acute respiratory symptoms including cough and dyspnea develop (Non-patent Literature 1), and when the condition is exacerbated, pneumonia develops. As of March 22, 2020, more than 290,000 patients infected with SARS-CoV-2 (COVID-19) and more than 12,000 deaths have been reported in the world. In Japan, 1,046 COVID-19 infection patients have been reported and of them, 36 deaths have been confirmed (Non-patent Literature 3).

As substances that may be effective for the treatment of COVID-19, baricitinib, lopinavir, ritonavir, darunavir, favipiravir (a pyrazine derivative), remdesivir, ribavirin, and the like have been reported (Non-patent Literature 4). However, no studies have systematically evaluated effects of combination use of two or more of these against coronavirus.

### Prior Art Documents

### Non-Patent Document

Non-Patent Literature 1: "Senryou Kagaku" (Dye Chemistry in Japanese), Yutaka Hosoda, Gihodo Co., Ltd., 1957, p.621 Wang C, Horby PW, Hayden FG, Gao GF. A novel coronavirus outbreak of global health concern. Lancet. 2020; 395:470-3.

Non-patent Literature 2: Coronaviridae Study Group of the International Committee on Taxonomy of Viruses. The species Severe acute respiratory syndrome-related coronavirus: classifying 2019-nCoV and naming it SARS-CoV-2. Nat Microbiol. 2020 Mar 2. PubMed PMID: 32123347.

Non-patent Literature 3: Coronavirus disease 2019 (COVID-19) situation reports - 62 (22 March 2020). [Internet]. Geneva: World Health Organization. Available from: https://www.who.int/docs/default-source/coronaviruse/situation-reports/20200322-sitrep-62-covid-19.pdf?sfvrsn=f7764c46 _2

Non-patent Literature 4: ACS Central Science (2020), 6(3), 315-331

### Summary of Invention

### Problem to be Solved by the Invention

An object of the present invention is to provide a novel combination of substances showing effects against coronavirus.

### Means to Solve the Problem

Under the above-described circumstances, the present inventors have found as a result of extensive studies that the antiviral activity against coronavirus is enhanced if a pyrazine derivative represented by general formula [1]

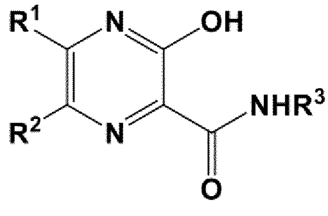

[1]

(wherein $R^1$ and $R^2$, identical or different, each represent a hydrogen atom or a halogen atom; and $R^3$ represents a hydrogen atom or an amino protecting group) or a salt thereof is used in combination with another therapeutic agent for

coronavirus infection, and accomplished the present invention.

Specifically, the present invention provides the following:

[1] A pharmaceutical composition for treating coronavirus infection, comprising a pyrazine derivative represented by the following general formula:

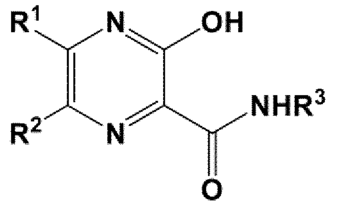 [1]

(wherein $R^1$ and $R^2$, identical or different, each represent a hydrogen atom or a halogen atom; and $R^3$ represents a hydrogen atom or an amino protecting group) or a salt thereof and one or more therapeutic agent for coronavirus infection selected from the following (1) to (19):

(1) Interferon;
(2) Nucleic acid analogue;
(3) Protease inhibitor;
(4) Furin convertase cleavage inhibitor;
(5) Reverse transcriptase inhibitor and/or other RNA polymerase inhibitor;
(6) Neuraminidase inhibitor;
(7) Angiotensin II receptor blocker;
(8) Endosome fusion inhibitor and/or endosome alkalinizer;
(9) AAK1, GAK, and clathrin A,B,C (endocytosis) inhibitor
(10) Hemagglutinin esterase inhibitor;
(11) Cytokine or inflammation inhibitor or modifier;
(12) Cathepsin B inhibitor;
(13) Cathepsin L inhibitor;
(14) Helicase nsp13 inhibitor;
(15) MBL2 gene agonist;
(16) TP53 inhibitor;
(17) Selective estrogen receptor modifier;
(18) Corticosteroid; and
(19) Amantadine, foscarnet, triazavirin, umifenovir, rapamycin, everolimus, nitazoxanide, tizoxanide, caraphenol A, ivermectin, VIR-2703, tocilizumab, bamlanivimab, etesebimab, kasiribimab/imdebimab, AZD7422, VIR-7831, VIR-7832, or BI 767551.

[2] The pharmaceutical composition according to [1], wherein the interferon is interferon α-2A, interferon α-2B, interferon α-n1, interferon α-n3, interferon β-1a, or interferon β-1b.

[3] The pharmaceutical composition according to [1], wherein the nucleic acid analogue is acyclovir, ganciclovir, ribavirin, or taribavirin.

[4] The pharmaceutical composition according to [1], wherein the protease inhibitor is indinavir, nelfinavir, saquinavir, camostat, lopinavir/ritonavir combination (brand name, Kaletra), epigallocatechin gallate, kaempferol-7-glucoside, mycophenolic acid, darunavir, mercaptopurine, disulfiram, nafamostat, or PF-07321332.

[5] The pharmaceutical composition according to [1], wherein the furin convertase cleavage inhibitor is tenofovir disoproxil, dolutegravir, boceprevir, andrographolide, luteolin, or baicalein.

[6] The pharmaceutical composition according to [1], wherein the reverse transcriptase inhibitor and/or other RNA polymerase inhibitor is remdesivir, sofosbuvir, dactinomycin, galidesivir, baloxavir marboxil, molnupiravir, sangibamycin, or AT-527.

[7] The pharmaceutical composition according to [1], wherein the neuraminidase inhibitor is oseltamivir or zanamivir.

[8] The pharmaceutical composition according to [1], wherein the angiotensin II receptor blocker is valsartan, telmisartan, losartan, irbesartan, azilsartan, olmesartan, or emodin.

[9] The pharmaceutical composition according to [1], wherein the endosome fusion inhibitor and/or endosome alkalinizer is baicalin, chloroquine, hydroxychloroquine, griffithsin, quinine, or lactoferrin.

[10] The pharmaceutical composition according to [1], wherein the AAK1, GAK, and clathrin A,B,C (endocytosis)

inhibitor is baricitinib, sunitinib, erlotinib, fedratinib, gefitinib, or silibinin.
[11] The pharmaceutical composition according to [1], wherein the hemagglutinin esterase inhibitor is 3,4-dichloroisocoumarin.
[12] The pharmaceutical composition according to [1], wherein the cytokine or inflammation inhibitor or modifier is ligustrazine, statin, melatonin, eplerenone, or methylprednisolone.
[13] The pharmaceutical composition according to [12], wherein the cathepsin B inhibitor is salvianolic acid B.
[14] The pharmaceutical composition according to [1], wherein cathepsin L inhibitor is MOL736, chelidocystatin, astaxanthin, curcumin, or vitamin D.
[15] The pharmaceutical composition according to [1], wherein the helicase nsp13 inhibitor is valsartan, bananin, iodobananin, vanillinbananin, eubananin, or silvestrol.
[16] The pharmaceutical composition according to [1], wherein the MBL2 gene agonist is β-glucan or vitamin A.
[17] The pharmaceutical composition according to [1], wherein the TP53 inhibitor is vitexin or gossypol.
[18] The pharmaceutical composition according to [1], wherein the selective estrogen receptor modifier is toremifene or equilin.
[19] The pharmaceutical composition according to [1], wherein the corticosteroid is ciclesonide or dexamethasone.
[20] The pharmaceutical composition according to [1], wherein the one or more therapeutic agent for coronavirus infection is a reverse transcriptase inhibitor and/or other RNA polymerase inhibitor.

The present invention further provides the following:

[A] A method for treating coronavirus infection, comprising administration of a pyrazine derivative represented by general formula [1]:

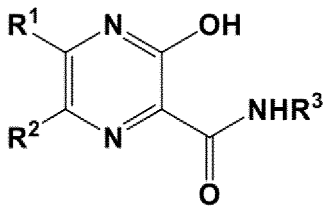 [1]

(wherein $R^1$ and $R^2$, identical or different, each represent a hydrogen atom or a halogen atom; and $R^3$ represents a hydrogen atom or an amino protecting group) or a salt thereof and another therapeutic agent for coronavirus infection.
[B] Use of a pyrazine derivative represented by general formula [1]:

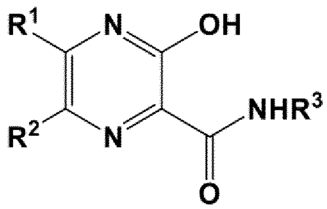 [1]

(wherein $R^1$ and $R^2$, identical or different, each represent a hydrogen atom or a halogen atom; and $R^3$ represents a hydrogen atom or an amino protecting group) or a salt thereof and another therapeutic agent for coronavirus infection to manufacture a therapeutic agent for coronavirus infection.
[C] A combination of a pyrazine derivative represented by general formula [1]

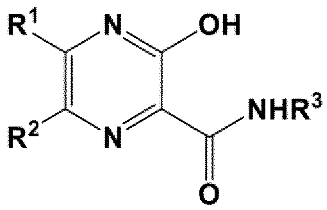 [1]

(wherein $R^1$ and $R^2$, identical or different, each represent a hydrogen atom or a halogen atom; and $R^3$ represents

a hydrogen atom or an amino protecting group) or a salt thereof and another therapeutic agent for coronavirus infection to use in the treatment of coronavirus infection.

**Advantageous Effects of Invention**

A therapeutic agent for coronavirus infection comprising a combination of a pyrazine derivative or a salt thereof and another therapeutic agent for coronavirus infection is useful for treatments such as therapeutic or prophylactic treatment of coronavirus infection.

**Embodiments for Carrying out the Invention**

The present invention will be described in detail below.

In this specification, a range of numerical values represented using "to" means a range including the numerical values before and after "to" as the minimum value and the maximum value, respectively.

The halogen atom means a fluorine atom, chlorine atom, bromine atom, or iodine atom. The $C_{1-6}$ alkyl group means a straight or branched $C_{1-6}$ alkyl group such as a methyl, ethyl, propyl, isopropyl, butyl, *sec*-butyl, isobutyl, *tert*-butyl, pentyl, isopentyl, 2-methylbutyl, 2-pentyl, 3-pentyl, or hexyl group.

The amino protecting groups include all groups that can be used as a usual amino protecting group, and examples thereof include groups listed in W. Greene et al., Protective Groups in Organic Synthesis Fifth Edition, pp.895-1193, 2014, John Wiley & Sons, Inc.

Specifically, examples thereof include an acyl group, alkyloxycarbonyl group, arylalkyloxycarbonyl group, aryloxycarbonyl group, arylalkyl group, alkoxyalkyl group, arylalkyloxyalkyl group, arylthio group, alkylsulfonyl group, arylsulfonyl group, dialkylaminoalkylidene group, arylalkylidene group, nitrogen-containing heterocyclic alkylidene group, cycloalkylidene group, diarylphosphoryl group, diarylalkylphosphoryl group, oxygenated heterocyclic alkyl group, and substituted silyl group.

Examples of a salt of the compound represented by general formula [1] include commonly known salts in a hydroxyl group. Examples thereof include salts with alkali metals such as sodium and potassium; salts with alkaline earth metals such as calcium and magnesium; ammonium salts; and salts with nitrogen-containing organic nucleotides such as trimethylamine, triethylamine, tributylamine, *N*-methylpiperidine, *N*-methylmorpholine, diethylamine, dicyclohexylamine, procaine, dibenzylamine, *N*-benzyl-β-phenethylamine, 1-efenamine, and *N,N*'-dibenzylethylenediamine. Preferred examples of salts include pharmacologically acceptable salts, and salts with sodium are more preferred.

In the compound represented by general formula [1], preferably, $R^1$ is a hydrogen atom; $R^2$ is a fluorine atom; and $R^3$ is a hydrogen atom. This compound is preferably T-705 (favipiravir).

Alternatively, in the compound represented by general formula [1], preferably, $R^1$ is a hydrogen atom; $R^2$ is a hydrogen atom; and $R^3$ is a hydrogen atom. Preferably, this compound is T-1105.

The compound represented by general formula [1] is manufactured by using methods known per se in combination. For example, it can be manufactured by the manufacturing methods described in International Publication No. WO 00/10569.

The therapeutic agent for coronavirus infection means a substance showing effect in therapeutic or prophylactic treatment of coronavirus infection by a certain mechanism of action, such as inhibition of proliferation of coronavirus in the host cell, inhibition of entry of coronavirus into the host cell, inhibition of egress of coronavirus out of the host cell, activation of the body's immune function, suppression of inflammation, and suppression of cytokine storm, and examples thereof include substances listed in the following (1) to (19):

(1) Interferon;
(2) Nucleic acid analogue;
(3) Protease inhibitor;
(4) Furin convertase cleavage inhibitor;
(5) Reverse transcriptase inhibitor and/or RNA polymerase inhibitor;
(6) Neuraminidase inhibitor;
(7) Angiotensin II receptor blocker;
(8) Endosome fusion inhibitor and/or endosome alkalinizer
(9) AAK1, GAK, or clathrin A,B,C (endocytosis) inhibitor;
(10) Hemagglutinin esterase inhibitor;
(11) Cytokine or inflammation inhibitor or modifier;
(12) Cathepsin B inhibitor;
(13) Cathepsin L inhibitor;
(14) Helicase nsp13 inhibitor;

(15) MBL2 gene agonist;
(16) TP53 inhibitor;
(17) Selective estrogen receptor modifier;
(18) Corticosteroid; and
(19) Other drugs.

Examples of the interferon include interferon α-2A, interferon α-2B, interferon α-n1, interferon α-n3, interferon β-1a, and interferon β-1b. It is sufficient to manufacture an interferon by using methods known per se in combination or to use a commercially available one.

Examples of the nucleic acid analogue include acyclovir, ganciclovir, ribavirin, and taribavirin. It is sufficient to manufacture a nucleic acid analogue by using methods known per se in combination or to use a commercially available one.

Examples of the protease inhibitor include indinavir, nelfinavir, saquinavir, camostat, lopinavir/ritonavir combination (brand name, Kaletra), epigallocatechin gallate, kaempferol-7-glucoside, mycophenolic acid, darunavir, mercaptopurine, disulfiram, nafamostat, and PF-07321332. It is sufficient to manufacture a protease inhibitor by using methods known per se in combination or to use a commercially available one.

Examples of the furin convertase cleavage inhibitor include tenofovir disoproxil, dolutegravir, boceprevir, andrographolide, luteolin, and baicalein. It is sufficient to manufacture a furin convertase cleavage inhibitor by using methods known per se in combination or to use a commercially available one.

Examples of the reverse transcriptase inhibitor and/or RNA polymerase inhibitor include remdesivir, sofosbuvir, dactinomycin, galidesivir, baloxavir marboxil, molnupiravir, sangibamycin and, AT-527. It is sufficient to manufacture a reverse transcriptase inhibitor and/or RNA polymerase inhibitor by using methods known per se in combination or to use a commercially available one.

Examples of the neuraminidase inhibitor include oseltamivir and zanamivir. It is sufficient to manufacture a neuraminidase inhibitor by using methods known per se in combination or to use a commercially available one.

Examples of the angiotensin II receptor blocker include valsartan, telmisartan, losartan, irbesartan, azilsartan, olmesartan, and emodin. It is sufficient to manufacture an angiotensin II receptor blocker by using methods known per se in combination or to use a commercially available one.

Examples of the endosome fusion inhibitor and/or endosome alkalinizer include baicalin, chloroquine, hydroxychloroquine, griffithsin, quinine, and lactoferrin. It is sufficient to manufacture an endosome fusion inhibitor and/or endosome alkalinizer by using methods known per se in combination or to use a commercially available one.

Examples of the AAK1, GAK, or clathrin A,B,C (endocytosis) inhibitor include baricitinib, sunitinib, erlotinib, fedratinib, gefitinib, and silibinin. It is sufficient to manufacture AAK1, GAK, or a clathrin A,B,C (endocytosis) inhibitor by using methods known per se in combination or to use a commercially available one.

Examples of the hemagglutinin esterase inhibitor include 3,4-dichloroisocoumarin. It is sufficient to manufacture a hemagglutinin esterase inhibitor by using methods known per se in combination or to use a commercially available one.

Examples of the cytokine or inflammation inhibitor or modifier include ligustrazine, statin, melatonin, eplerenone, and methylprednisolone. It is sufficient to manufacture a cytokine or inflammation inhibitor or modifier by using methods known per se in combination or to use a commercially available one.

Examples of the cathepsin B inhibitor include salvianolic acid B. It is sufficient to manufacture a cathepsin B inhibitor by using methods known per se in combination or to use a commercially available one.

Examples of the cathepsin L inhibitor include MOL736, chelidocystatin, astaxanthin, curcumin, and vitamin D. It is sufficient to manufacture a cathepsin L inhibitor by using methods known per se in combination or to use a commercially available one.

Examples of the helicase nsp13 inhibitor include valsartan, bananin, iodobananin, vanillinbananin, eubananin, and silvestrol. It is sufficient to manufacture a helicase nsp13 inhibitor by using methods known per se in combination or to use a commercially available one.

Examples of the MBL2 gene agonist include β-glucan and vitamin A. It is sufficient to manufacture an MBL2 gene agonist by using methods known per se in combination or to use a commercially available one.

Examples of the TP53 inhibitor include vitexin and gossypol. It is sufficient to manufacture a TP53 inhibitor by using methods known per se in combination or to use a commercially available one.

Examples of the selective estrogen receptor modifier include toremifene and equilin. It is sufficient to manufacture a selective estrogen receptor modifier by using methods known per se in combination or to use a commercially available one.

Examples of the corticosteroid include ciclesonide and dexamethasone. It is sufficient to manufacture a corticosteroid by using methods known per se in combination or to use a commercially available one.

Examples of the other drugs include amantadine, foscarnet, triazavirin, umifenovir, rapamycin, everolimus, nitazoxanide, tizoxanide, caraphenol A, ivermectin, VIR-2703, tocilizumab, bamlanivimab, etesebimab, kasiribimab/imdebimab, AZD7422, VIR-7831, VIR-7832, and BI 767551. It is sufficient to manufacture amantadine, foscarnet, triazavirin, umifenovir, rapamycin, everolimus, nitazoxanide, tizoxanide, caraphenol A, ivermectin, VIR-2703, tocilizumab, bamlanivimab, etesebimab, kasiribimab/imdebimab, AZD7422, VIR-7831, VIR-7832, and BI 767551. by using methods known per se in combination or to use commercially available ones.

In the present invention, a pyrazine derivative and another therapeutic agent for coronavirus infection are used in combination. The combination encompasses an embodiment of administering a pyrazine derivative and another therapeutic agent for coronavirus infection simultaneously, separately, or in a specific order (combination use) and an embodiment as a mixture (a combination drug).

Specifically, the combination use does not mean only an embodiment of administering a pyrazine derivative or a salt thereof and another therapeutic agent for coronavirus infection at one time, but also encompasses an embodiment of administering a pyrazine derivative or a salt thereof and another therapeutic agent for coronavirus infection during one dosing regimen. The routes of administration of a pyrazine derivative or a salt thereof and another therapeutic agent for coronavirus infection may be identical to or different from each other.

It is sufficient to select an amount ratio of a pyrazine derivative or a salt thereof and another therapeutic agent for coronavirus infection, such that both compounds exhibit their effects additively or synergistically, and an amount ratio in which both compounds can exhibit their effects synergistically is preferred. An amount ratio (molar ratio) of a pyrazine derivative or a salt thereof and another therapeutic agent for coronavirus infection is preferably 1:500 to 500:1, more preferably 1:200 to 200:1, yet more preferably 1:50 to 50: 1, and further preferably 1:10 to 10:1.

When the pyrazine derivative or a salt thereof and another therapeutic agent for coronavirus infection of the present invention is used, pharmaceutical aids usually used for formulation, such as excipients, carriers, and diluents, may be suitably added, and these compounds can be formulated into a form such as tablet, capsule, powder, syrup, granule, pill, suspension, emulsion, solution, powder formulation, suppository, eye drop, nasal drop, ear drop, patch, ointment, or injection by a usual method.

When a pyrazine derivative or a salt thereof and another therapeutic agent for coronavirus infection are used as a combination drug, it is sufficient to mix and homogenize them during the process of the above-mentioned formulation to obtain a suitable formulations.

The route of administration of the therapeutic agent for coronavirus infection of the present invention is not particularly limited, and it can be administered intravenously, orally, intramuscularly, subcutaneously, by inhalation, by aerosolization, or other routes of administration. The pyrazine derivative or a salt thereof may be administered with another therapeutic agent for coronavirus infection simultaneously or in a specific order.

The administration method, dose, and number of doses of the pyrazine derivative or a salt thereof of the present invention can be selected suitably depending on the patient's age, body weight, and symptoms. The adult dose for oral or parenteral (e.g., injection, drip infusion, and rectal administration) administration of a pyrazine derivative or a salt thereof, an active ingredient, is usually 10 to 50,000 mg or preferably 200 to 24,000 mg, which can be administered once a day (QD) or divided into a several doses a day. It is typical that administration is preferably repeated, as necessary, while being monitored. Specifically, for adults, it is sufficient to be administered at 100 to 6,000 mg QD, twice a day (BID), three times a day (TTD), or four times a day (QID) on Day 1 and at 100 to 6,000 mg QD, BID, TID, or QID on Day 2 and thereafter. Preferably, the dose on Day 1 (a loading dose) is higher than the dose on Day 2 and thereafter, and the dose on Day 1 is 1.5 times or higher, two times or higher, three times or higher, four times or higher, or five times or higher than the dose on Day 2 and thereafter.

More specifically, it is sufficient to be administered at 1,000 to 5,000 mg QD, BID, TID, or QID on Day 1 and at 400 to 2,400 mg QD, BID, TID, or QID on Day 2 and thereafter. The adult dosage is preferably 1,600 mg BID on Day 1 and 600 mg BID on Day 2 and thereafter, 1,800 mg BID on Day 1 and 800 mg BID on Day 2 and thereafter, or 1,800 mg BID on Day 1 and 1000 mg BID on Day 2 and thereafter, more preferably 1,800 mg BID on Day 1 and 800 mg BID on Day 2 and thereafter.

The second dose is administered preferably with an interval of at least 4 hours, more preferably with an interval at least 6 hours, after the first dose.

The duration of administration is suitably determined depending on change over time in symptoms, and for example, can be selected from up to 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, and 31 days and 1month, 2 months, and 3 months. Up to 10 days, 13 days, 14 days, and 22 days are preferred, and up to 14 days is more preferred. For prophylactic treatment, it is recommended to continue treatment during the period in which the infection risk is high.

The half maximal effective concentration (EC50) of T-705 included in the pyrazine derivatives of the present invention against SARS-CoV-2 was 61.88 μM in a study using Vero E6 cells (Wang M, Cao R, Zhang L, Yang X, Liu J, Xu M, et al. Remdesivir and chloroquine effectively inhibit the recently emerged novel coronavirus [2019-nCoV] in vitro. Cell Res. 2020; 30:269-71.). Since this concentration is equivalent to 9.72 μg/mL, the pyrazine derivative or a salt thereof is preferably administered, such that a blood concentration will be 9.72 μg/mL or higher. However, this shall not apply

to the embodiments of the present invention that can be expected to have a synergistic effect.

The administration method, dose, and number of doses of the other therapeutic agent for coronavirus infection of the present invention can be suitably selected depending on the patient's age, body weight, and symptoms. They are preferably selected on the basis of known clinical study protocols and results. Specific examples thereof include the following:
When hydroxychloroquine is used, it is sufficient to be administered, for example, at 800 mg BID on Day 1 and at 400 mg BID on Day 2 and thereafter for up to Day 10 or at 1,200 mg divided into QID for 5 to 10 days; as a sulfate, at 800 mg BID on Day 1 and at 400 mg BID on Days 2 to 5 or at 600 mg BID on Day 1 and at 400 mg BID on Days 2 to 7.

When a lopinavir/ritonavir combination is used, it is sufficient to administer, for example, lopinavir at 200 mg/ritonavir at 100 mg BID for 7 to 10 days, lopinavir at 400 mg/ritonavir at 100 mg QD for up to 14 days, lopinavir at 400 mg/ritonavir at 100 mg BID for up to 14 days, or lopinavir at400 mg/ritonavir at 200 mg BID for 5 to 14 days.

When ciclesonide is used, it is sufficient to administer, for example, ciclesonide at 320 μg for inhalation every 12 hours for up to 14 days or ciclesonide at 200 μg for inhalation every 12 hours for up to 14 days.

When remdesivir is used, it is sufficient to be administered, for example, at 200 mg QD on Day 1 and at 100 mg QD on Days 2 to 10, which can be extended to Day 13, at 200 mg QD on Day 1 and at 100 mg QD on Days 2 to 5, or at 400 mg BID on Day 1 and at 400 mg QD for 2 to 11 days; and at 5 mg/kg QD on Day 1 and at 2.5 mg/kg QD for Days 2 to 10, which can be extended to Day 13, for children with body weight lower than 40 kg.

When molnupiravir is used, it is sufficient to be administered, for example, at 200 mg, 400 mg, 600 mg, 800 mg, 1000 mg, 1200 mg, 1400 mg, 1600 mg, 1800 mg, or 2000 mg BID for 5 days. It is administrated preferably at 800 mg BID for 5 days.

The therapeutic agent for coronavirus infection of the present invention is useful for treatments such as therapeutic or prophylactic treatment of coronavirus infection.

The term "coronavirus" used in this specification means an RNA virus belonging to the family *coronaviridae,* the order *nidovirales.* Coronavirus infection is an infectious disease caused by coronavirus. The term "novel coronavirus" means a newly identified and reported novel coronavirus among coronaviruses, and examples thereof include SARS-CoV-2 reported at the end of 2019. The infectious disease caused by SARS-CoV-2 is also called COVID-19. The term "novel coronavirus" naturally encompasses mutants and variants of the known coronavirus. The term "coronavirus infection" naturally encompasses infectious diseases caused by the novel coronavirus, including COVID-19.

## Examples

The present invention will be described using examples, but the present invention is not limited to these.

Test example 1

A test was performed using a viral infection cell model to evaluate the effect of combination use of a pyrazine derivative and remdesivir. Specifically, the antiviral effect was evaluated by determining the cytopathic effect (CPE). In addition to the CPE assay, the antiviral effect can be evaluated by a viral antigen-antibody method or by real-time polymerase chain reaction (PCR) of virus RNA extracted from a virus culture supernatant.

T-705 was selected as a pyrazine derivative. Remdesivir was selected as another therapeutic agent for coronavirus infection. Novel coronavirus (SARS-CoV-2) was selected as an RNA virus.

(1) Vero E6 cell culture

African green monkey kidney Vero E6 cells that were subcultured in a 10% fetal bovine serum-added Eagle minimum essential medium (MEM) containing 60 μg/mL kanamycin (Eagle MEM/kanamycin medium) at 37°C under the 5% carbon dioxide condition were removed from the culture broth by the trypsin-ethylenediaminetetraacetic acid method, and a suspension containing $2 \times 10^4$ cells per 100 μL of the medium was prepared and seeded on a 96 well plate. The Vero E6 cells were cultured overnight at 37°C under the 5% carbon dioxide condition and obtained as a monolayer.

(2) SARS-CoV-2 infection and addition of drugs

A 2% fetal bovine serum-added Eagle MEM/kanamycin medium was used as a test medium. The culture supernatant containing Vero E6 cells obtained in (1) was removed, the following (A) or (B) was added to each well, and the mixture was cultured at 37°C for 2 hours under the 5% carbon dioxide condition:

(A) 100 μL of SARS-CoV-2 solution prepared with the test medium to obtain a final multiplicity of infection of 0.02

(B) 100 μL of 0.5% DMSO-containing test medium that contained a combination of T-705 and remdesivir at concentrations two-fold the specified concentrations for each combination of the specified concentrations:

Specified concentrations (μM) of T-705:
0, 0.1, 0.3, 1, 3, 10, 30, 100, 300, 1000
Specified concentrations (μM) of remdesivir:
0, 0.1, 0.3, 1, 3, 10, 30, 100

At 2 hours after infection, the culture supernatant was removed from each well, and 100 μL of 0.5% DMSO-containing test medium that contained a combination of T-705 and remdesivir at concentrations one-fold the specified concentration for each combination of the specified concentrations were added. After the drugs were added, the cells were cultured at 37°C for 2 days under the 5% carbon dioxide condition.

(3) Determination of cytopathic effect (CPE)

The CPE associated with proliferation of SARS-CoV-2 was determined by the following method.
After completion of culture, 25 μL of 100% formalin solution was added to each well to inactivate virus and immobilize cells. The mixture was left stand at room temperature for 2 hours or longer, the aqueous solution was removed, cells were gently washed with water, and then 50 μL of0.02% methylene blue solution was added to each well, and the mixture was left stand at room temperature for one hour. The aqueous solution was removed, and cells were gently washed with water and then air-dried. Then, absorbance (660 nm) was measured using a microplate reader (Tecan). For a non-infected control, 100 μL of the test medium was added instead of the SARS-CoV-2 solution, the same operations as for the test group were performed, and the absorbance was measured.
A test was performed for two plates (one patient per plate) (eight patients for infected controls and non-infected controls). The value obtained by subtracting the absorbance of the infected control from the absorbance of the non-infected control was used as the value indicating complete inhibition of viral proliferation, and the CPE inhibition rate was calculated for each test by the following formula:

$$\text{CPE inhibition rate} = 100 \times ([\text{absorbance after monotherapy or combination use}) - (\text{absorbance of infected control}]) / ([\text{absorbance of non-infected control}) - (\text{absorbance of infected control}])$$

Microsoft Office Excel 2016 FORECAST function (linear regression) was used to calculate 50% CPE inhibitory concentration.
The 50% CPE inhibitory concentration was confirmed when T-705 and remdesivir were used in combination at concentrations lower than their respective 50% inhibitory concentrations ($IC_{50}$) of monotherapy.
In addition to remdesivir, the same test as described above was performed for the therapeutic agents for coronavirus infection listed in Table 1. The $IC_{50}$ was confirmed when T-705 and remdesivir were used in combination at concentrations lower than their respective $IC_{50}$ of monotherapy.

[Table 1-1]

| No. | Name |
|---|---|
| 1 | Interferon α-2A |
| 2 | Interferon α-2B |
| 3 | Interferon α-n1 |
| 4 | Interferon α-n3 |
| 5 | Interferon β-1a |
| 6 | Interferon β-1b |
| 7 | Acyclovir |
| 8 | Ganciclovir |

(continued)

| No. | Name |
|---|---|
| 9 | Ribavirin |
| 10 | Taribavirin |
| 11 | Indinavir |
| 12 | Nelfinavir |
| 13 | Saquinavir |
| 14 | Camo stat |
| 15 | Lopinavir/ritonavir combination (brand name, Kaletra) |
| 16 | Epigallocatechin gallate |
| 17 | Kaempferol-7-glucoside |
| 18 | Mycophenolic acid |
| 19 | Darunavir |
| 20 | Mercaptopurine |
| 21 | Disulfiram |
| 22 | Tenofovir disoproxil |
| 23 | Dolutegravir |
| 24 | Boceprevir |
| 25 | Andrographolide |
| 26 | Luteolin |
| 27 | Baicalein |
| 28 | Sofosbuvir |
| 29 | Dactinomycin |
| 30 | Galidesivir |
| 31 | Baloxavir marboxil |
| 32 | Oseltamivir |
| 33 | Zanamivir |
| 34 | Valsartan |
| 35 | Telmisartan |

[Table 1-2]

| | |
|---|---|
| 36 | Lo sartan |
| 37 | Irbesartan |
| 38 | Azilsartan |
| 39 | Olmesartan |
| 40 | Emodin |
| 41 | Baicalin |
| 42 | Chloroquine |
| 43 | Hydroxychloroquine |

(continued)

| | |
|---|---|
| 44 | Griffithsin |
| 45 | Quinine |
| 46 | Lactoferrin |
| 47 | Baricitinib |
| 48 | Sunitinib |
| 49 | Erlotinib |
| 50 | Fedratinib |
| 51 | Gefitinib |
| 52 | Silibinin |
| 53 | 3,4-Dichloroisocoumarin |
| 54 | Ligustrazine |
| 55 | Statin |
| 56 | Melatonin |
| 57 | Eplerenone |
| 58 | Methylprednisolone |
| 59 | Salvianolic acid B |
| 60 | MOL736 |
| 61 | Chelidocystatin |
| 62 | Astaxanthin |
| 63 | Curcumin |
| 64 | Vitamin D |
| 65 | Valsartan |
| 66 | Bananin |
| 67 | Iodobananin |
| 68 | Vanillinbananin |
| 69 | Eubananin |
| 70 | Silvestrol |
| 71 | β-glucan |

[Table 1-3]

| | |
|---|---|
| 72 | Vitamin A |
| 73 | Vitexin |
| 74 | Gossypol |
| 75 | Toremifene |
| 76 | Equilin |
| 77 | Ciclesonide |
| 78 | Amantadine |
| 79 | Foscarnet |

(continued)

| | |
|---|---|
| 80 | Triazavirin |
| 81 | Umifenovir |
| 82 | Rapamycin |
| 83 | Everolimus |
| 84 | Nitazoxanide |
| 85 | Tizoxanide |
| 86 | Caraphenol A |

[Table 1-4]

| | |
|---|---|
| 87 | Dexamethasone |
| 88 | Nafamostat |
| 89 | Ivermectin |
| 90 | Molnupiravir |
| 91 | Sangibamycin |
| 92 | AT-527 |
| 93 | PF-07321332 |
| 94 | VIR-2703 |
| 95 | Tocilizumab |
| 96 | Bamlanivimab |
| 97 | Etesebimab |
| 98 | Kasiribimab/Imdebimab |
| 99 | AZD7422 |
| 100 | VIR-7831 |
| 101 | VIR-7832 |
| 102 | BI 767551 |

Test example 2

T-705 was selected as a pyrazine derivative. Remdesivir was selected as another therapeutic agent for coronavirus infection. Human coronavirus (HCoV-OC43) responsible for common cold, which belongs to the genus *betacoronavirus* as with the novel coronavirus, was selected as an RNA virus.

(1) Culture of BHK-21 cells

Hamster kidney BHK-21 cells that were subcultured in a 10% fetal bovine serum-added EMEM containing 60 μg/mL kanamycin (EMEM/kanamycin medium) at 37°C under the 5% carbon dioxide condition were removed from the culture broth by the trypsin-ethylenediaminetetraacetic acid method, then a suspension containing $4 \times 10^4$ cells per 100 μL in the medium was prepared and seeded on a 96 well plate. The BHK-21 cells were cultured overnight at 37°C under the 5% carbon dioxide condition and obtained as a monolayer.

(2) HCoV-OC43 infection and addition of drugs

A 2% fetal bovine serum-added EMEM/kanamycin medium was used as a test medium. The culture supernatant containing BHK-21 cells obtained in (1) was removed, the following (A) to (C) were added:

(A) 100 μL of the test medium
(B) 50 μL of HCoV-OC43 solution prepared with the test medium to obtain a final infectivity titer of $4.0\times10^3$ $TCID_{50}$
(C) 50 μL of 1% DMSO containing test medium that contained a combination of T-705 and remdesivir at concentrations four-fold the specified concentrations for each combination of the specified concentrations:

| | |
|---|---|
| Specified concentration (μM) of T-705: | 0, 1, 10, 100, 1000 |
| Specified concentration (μM) of remdesivir: | 0, 0.1, 0.3 |

After the drugs were added, the cells were cultured at 33°C for 3 to 4 days under the 5% carbon dioxide condition.

(3) Determination of cytopathic effect (CPE)

The CPE associated with proliferation of HCoV-OC43 was assessed by the following method.
After completion of culture, 50 μL of 100% formalin solution was added to each well to inactivate virus and immobilized cells. The mixture was left stand at room temperature for 2 hours or longer, the aqueous solution was removed, and cells were gently washed with water, and then 50 μL of 0.02% methylene blue solution was added to each well, and the mixture was left stand at room temperature for one hour. The aqueous solution was removed, and cells were gently washed with water and then air-dried. Then, absorbance at 660 nm was measured using a microplate reader (Tecan). For a non-infected control, 50 μL of the test medium was added instead of the HCoV-OC43 solution, the same operations as for the test group were performed, and the absorbance was measured.
A test was performed for two plates (one patient per plate) (six patients for infected controls and non-infected controls). The value obtained by subtracting the absorbance of the infected control from the absorbance of the non-infected control was used as the value indicating complete inhibition of viral proliferation, and the CPE inhibition rate was calculated for each test by the following formula:

$$\text{CPE inhibition rate} = 100 \times ([\text{absorbance after monotherapy or combination use}) - (\text{absorbance of infected control}]) / ([\text{absorbance of non-infected control}) - (\text{absorbance of infected control}])$$

Microsoft Office Excel 2016 FORECAST function (linear regression) was used to calculate 50% CPE inhibitory concentration.
The CPE inhibition rates on single use or combination use of drug(s) were compared. A CPE inhibition rate on single use of 1000 μM of T-705 was 79%, and a CPE inhibition rate on each of the single use of 0.1 μM and 0.3 μM of remdesivir was 51% and 80%, respectively. In contrast, a CPE inhibition rate on each of combination use of 0.1 μM o T-705 and remdesivir and 0.3 μM of those was 92% and 102%, respectively.
The combination use increased the CPE inhibition rate compared to each of single use of T-705 and remdesivir, and almost 100% of CPE inhibition effect thereof was confirmed. Also, on the basis of this result, it is considered that drugs having a mechanism of action similar to remdesivir, namely reverse transcriptase inhibitors and/or RNA polymerase inhibitors (for example, molnupiravir) are also effective in combination with T-705.

## Industrial Applicability

A therapeutic agent for coronavirus infection comprising a combination of a pyrazine derivative or a salt thereof and another therapeutic agent for coronavirus infection is useful in the field of pharmaceutical industry.

## Claims

1. A pharmaceutical composition for treating coronavirus infection, comprising a pyrazine derivative represented by the following general formula:

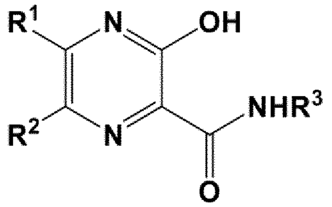 [1]

(wherein $R^1$ and $R^2$, identical or different, each represent a hydrogen atom or a halogen atom; and $R^3$ represents a hydrogen atom or an amino protecting group) or a salt thereof and one or more therapeutic agents for coronavirus infection selected from the following (1) to (19):

(1) Interferon;
(2) Nucleic acid analogue;
(3) Protease inhibitor;
(4) Furin convertase cleavage inhibitor;
(5) Reverse transcriptase inhibitor and/or other RNA polymerase inhibitor;
(6) Neuraminidase inhibitor;
(7) Angiotensin II receptor blocker;
(8) Endosome fusion inhibitor and/or endosome alkalinizer;
(9) AAK1, GAK, or clathrin A,B,C (endocytosis) inhibitor;
(10) Hemagglutinin esterase inhibitor;
(11) Cytokine or inflammation inhibitor or modifier;
(12) Cathepsin B inhibitor;
(13) Cathepsin L inhibitor;
(14) Helicase nsp13 inhibitor;
(15) MBL2 gene agonist;
(16) TP53 inhibitor;
(17) Selective estrogen receptor modifier;
(18) Corticosteroid; and
(19) Amantadine, foscarnet, triazavirin, umifenovir, rapamycin, everolimus, nitazoxanide, tizoxanide, caraphenol A, ivermectin, VIR-2703, tocilizumab, bamlanivimab, etesebimab, kasiribimab/imdebimab, AZD7422, VIR-7831, VIR-7832, or BI 767551.

2. The pharmaceutical composition according to claim 1, wherein the interferon is interferon α-2A, interferon α-2B, interferon α-n1, interferon α-n3, interferon β-1a, or interferon β-1b.

3. The pharmaceutical composition according to claim 1, wherein the nucleic acid analogue is acyclovir, ganciclovir, ribavirin or taribavirin.

4. The pharmaceutical composition according to claim 1, wherein the protease inhibitor is indinavir, nelfinavir, saquinavir, camostat, lopinavir/ritonavir combination (brand name, Kaletra), epigallocatechin gallate, kaempferol-7-glucoside, mycophenolic acid, darunavir, mercaptopurine, disulfiram, nafamostat, or PF-07321332.

5. The pharmaceutical composition according to claim 1, wherein the furin convertase cleavage inhibitor is tenofovir disoproxil, dolutegravir, boceprevir, andrographolide, luteolin, or baicalein.

6. The pharmaceutical composition according to claim 1, wherein the reverse transcriptase inhibitor and/or other RNA polymerase inhibitor is remdesivir, sofosbuvir, dactinomycin, galidesivir, baloxavir marboxil, molnupiravir, sangibamycin, or AT-527.

7. The pharmaceutical composition according to claim 1, wherein the neuraminidase inhibitor is oseltamivir or zanamivir.

8. The pharmaceutical composition according to claim 1, wherein the angiotensin II receptor blocker is valsartan, telmisartan, losartan, irbesartan, azilsartan, olmesartan, or emodin.

9. The pharmaceutical composition according to claim 1, wherein the endosome fusion inhibitor and/or endosome alkalinizer is baicalin, chloroquine, hydroxychloroquine, griffithsin, quinine, or lactoferrin.

**10.** The pharmaceutical composition according to claim 1, wherein the AAK1, GAK, or clathrin A, B, C (endocytosis) inhibitor is baricitinib, sunitinib, erlotinib, fedratinib, gefitinib, or silibinin.

**11.** The pharmaceutical composition according to claim 1, wherein the hemagglutinin esterase inhibitor is 3,4-dichloroisocoumarin.

**12.** The pharmaceutical composition according to claim 1, wherein the cytokine or inflammation inhibitor or modifier is ligustrazine, statin, melatonin, eplerenone, or methylprednisolone.

**13.** The pharmaceutical composition according to claim 1, wherein the cathepsin B inhibitor is salvianolic acid B.

**14.** The pharmaceutical composition according to claim 1, wherein the cathepsin L inhibitor is MOL736, chelidocystatin, astaxanthin, curcumin, or vitamin D.

**15.** The pharmaceutical composition according to claim 1, wherein the helicase nsp13 inhibitor is valsartan, bananin, iodobananin, vanillinbananin, eubananin, or silvestrol.

**16.** The pharmaceutical composition according to claim 1, wherein the MBL2 gene agonist is β-glucan or vitamin A.

**17.** The pharmaceutical composition according to claim 1, wherein the TP53 inhibitor is vitexin or gossypol.

**18.** The pharmaceutical composition according to claim 1, wherein the selective estrogen receptor modifier is toremifene or equilin.

**19.** The pharmaceutical composition according to claim 1, wherein the corticosteroid is ciclesonide or dexamethasone.

**20.** The pharmaceutical composition according to claim 1, wherein the one or more therapeutic agent for coronavirus infection is a reverse transcriptase inhibitor and/or other RNA polymerase inhibitor.

# INTERNATIONAL SEARCH REPORT

International application No.
PCT/JP2021/016737

A. CLASSIFICATION OF SUBJECT MATTER
see extra sheet

According to International Patent Classification (IPC) or to both national classification and IPC

B. FIELDS SEARCHED

Minimum documentation searched (classification system followed by classification symbols)
see extra sheet

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | CAI, Q. et al., "Experimental Treatment with Favipiravir for COVID-19: An Open-Label Control Study.", Engineering, March 2020, 6, pp. 1192-1198 abstract, page 1193, left column, paragraph [0003], page 1193, right column, paragraph [0002], table 2 | 1, 2<br>1-20 |
| Y | CHOY, K. T. et al., "Remdesivir, lopinavir, emetine, and homoharringtonine inhibit SARS-CoV-2 replication in vitro.", Antiviral Research, 03 April 2020, 178, 104786, pp. 1-5 page 1, right column, paragraph [0001], fig. 1 | 1-4, 6, 9, 20 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 June 2021 (25.06.2021) | 20 July 2021 (20.07.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## INTERNATIONAL SEARCH REPORT

International application No. PCT/JP2021/016737

C (Continuation). DOCUMENTS CONSIDERED TO BE RELEVANT

| Category* | Citation of document, with indication, where appropriate, of the relevant passages | Relevant to claim No. |
|---|---|---|
| Y | BECK, B. R. et al., "Predicting commercially available antiviral drugs that may act on the novel coronavirus (SARS-CoV-2)through a drug-target interaction deep learning model.", Computational and Structural Biotechnology Journal, March 2020, 18, pp. 784-790 abstract, table 1 | 1, 4, 5 |
| Y | WILLIAMSON, B. N. et al., "Clinical benefit of remdesivir in rhesus macaques infected with SARS-CoV-2.", bioRxiv, 22 April 2020, pp. 1-21, DOI: 10.1101/2020.04.15.043166 abstract, fig. 4, 5 | 1, 6, 20 |
| Y | WANG, M. et al., "Remdesivir and chloroquine effectively inhibit the recently emerged novel coronavirus (2019-nCoV) in vitro.", Cell Research, February 2020, 30(3), pp. 269-271 fig. 1 | 1, 6, 9, 20 |
| Y | GREIN, J. et al., "Compassionate Use of Remdesivir for Patients with Severe Covid-19.", N. Engl. J. Med., 10 April 2020, pp. 1-10, doi: 10.1056/NEJMoa2007016. abstract, page 3, right column, the last paragraph to page 5, left column, paragraph [0002] | 1, 6, 20 |
| Y | JP 2016-512833 A (GEMMUS PHARMA INC.) 09 May 2016 (2016-05-09) claims 9, 10, 28 | 1, 7 |
| Y | JP 2017-524339 A (REGENERON PHARMACEUTICALS, INC.) 31 August 2017 (2017-08-31) claim 1, paragraph [0080] | 1, 7 |
| Y | JP 2008-540600 A (IMBA-INSTITUT FÜR MOLEKULARE BIOTECHNOLOGIE GMBH) 20 November 2008 (2008-11-20) claims 1, 4 | 1, 8, 15 |
| Y | JP 2012-512883 A (THE REGENTS OF THE UNIVERSITY OF CALIFORNIA) 07 June 2012 (2012-06-07) example 3 | 1, 10 |
| Y | VLASAK, R. et al., "INFLUENZA C VIRUS ESTERASE: ANALYSIS OF CATALYTIC SITE, INHIBITION, AND POSSIBLE FUNCTION.", Journal of Virology, 1989, 63(5), pp. 2056-2062 abstract, table 3 | 1, 11 |
| Y | MATSUYAMA, S. et al., "The inhaled corticosteroid ciclesonide blocks coronavirus RNA replication by targeting viral NSP15.", bioRxiv, March 2020, doi: https://doi.org/10.1101/2020.03.11.987016 in particular, fig. 2 | 1, 12, 19 |

Form PCT/ISA/210 (continuation of second sheet) (January 2015)

INTERNATIONAL SEARCH REPORT

International application No.
PCT/JP2021/016737

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2015-28087 A (REVALESIO CORPORATION) 12 February 2015 (2015-02-12) claims 1, 7 | 1, 12, 19 |
| Y | JP 2016-539944 A (BAYLOR COLLEGE OF MEDICINE) 22 December 2016 (2016-12-22) claim 1, paragraph [0092] | 1, 12, 19 |
| Y | JP 2002-518455 A (GEORGETOWN UNIVERSITY) 25 June 2002 (2002-06-25) example 6 | 1, 13 |
| Y | JP 2015-525221 A (GILEAD SCIENCES, INC.) 03 September 2015 (2015-09-03) paragraph [0268] | 1, 14 |
| Y | JP 2016-65037 A (NIKKEN SOHONSHA CORPORATION) 28 April 2016 (2016-04-28) paragraphs [0016], [0045]-[0047] | 1, 16 |
| Y | JP 2017-178913 A (EUGLENA CO., LTD.) 05 October 2017 (2017-10-05) paragraph [0039] | 1, 17 |
| Y | JOHANSEN, L. M. et al., "FDA-Approved Selective Estrogen Receptor Modulators Inhibit Ebola Virus Infection.", Science Translational Medicine, 2013, 5(190), 190ra79, pp. 1-13 abstract, fig. 4 | 1, 18 |
| Y | XU, X.et al., "Effective treatment of severe COVID-19 patients with Tocilizumab.", ChinaXiV, March 2020, vol. 21, pp. 1-10 in particular, table 2 | 1 |
| P, A | WANG, D. et al., "An overview of the safety, clinical application and antiviral research of the COVID-19 therapeutics.", Journal of Infection and Public Health, July 2020, 13, pp. 1405-1414 entire text | 1-20 |
| P, A | SANDERS, J. M. et al., "Pharmacologic treatments for coronavirus disease 2019(COVID-19).", JAMA, May 2020, 323(18), pp. 1824-1836 entire text | 1-20 |
| P, A | WO 2020/138067 A1 (FUJIFILM TOYAMA CHEMICAL CO., LTD.) 02 July 2020 (2020-07-02) | 1-20 |

Form PCT/ISA/210 (continuation of second sheet) (January 2015)

**INTERNATIONAL SEARCH REPORT**

International application No.
PCT/JP2021/016737

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
PCT/JP2021/016737

| Patent Documents referred in the Report | Publication Date | Patent Family | Publication Date |
|---|---|---|---|
| JP 2016-512833 A | 09 May 2016 | WO 2014/151085 A1<br>claims 9, 10, 28<br>US 2014/0275237 A1<br>EP 2968362 A1<br>CA 2906528 A1<br>AU 2014235346 A<br>KR 10-2015-0132440 A<br>CN 105555276 A | |
| JP 2017-524339 A | 31 Aug. 2017 | WO 2015/179535 A1<br>claim 1, paragraph [0091]<br>US 2015/0337029 A1<br>EP 3145539 A1<br>AU 2015264189 A<br>KR 10-2017-0005133 A<br>CA 2949438 A1<br>CN 106414496 A | |
| JP 2008-540600 A | 20 Nov. 2008 | WO 2006/122819 A1<br>claims 1, 4<br>US 2008/0159962 A1<br>EP 1723962 A1<br>CA 2608376 A1<br>AU 2006249084 A | |
| JP 2012-51883 A | 07 Jun. 2012 | US 2011/0236349 A1<br>example 3<br>WO 2010/123527 A2<br>EP 2373329 A1<br>CA 2744947 A1<br>AU 2009344851 A | |
| JP 2015-28087 A | 12 Feb. 2015 | US 2009/0274730 A1<br>claims 1, 15<br>WO 2009/055729 A1<br>EP 2207669 A1<br>AU 2008316794 A<br>CA 2703714 A1<br>CN 101910412 A | |
| JP 2016-539944 A | 22 Dec. 2016 | WO 2015/080973 A1<br>claim 1, paragraph [0092]<br>US 2016/0376321 A1<br>KR 10-2016-0079920 A<br>CN 105934441 A | |
| JP 2002-518455 A | 25 Jun. 2002 | WO 1999/066942 A1<br>example 6<br>US 6043276 A<br>EP 1089747 A1<br>CA 2335956 A1<br>CN 1312722 A<br>KR 10-2001-0071580 A | |

Form PCT/ISA/210 (patent family annex) (January 2015)

**INTERNATIONAL SEARCH REPORT**
Information on patent family members

International application No. PCT/JP2021/016737

| Patent Documents referred in the Report | Publication Date | Patent Family | Publication Date |
|---|---|---|---|
| JP 2015-525221 A | 03 Sep. 2015 | WO 2013/185093 A1<br>page 52, line 13<br>US 2015/0361132 A1<br>EP 2861601 A1<br>CA 2875690 A1<br>AU 2013270670 A<br>KR 10-2015-0040267 A<br>CN 104781261 A | |
| JP 2016-65037 A | 28 Apr. 2016 | (Family: none) | |
| JP 2017-178913 A | 05 Oct. 2017 | WO 2017/170962 A1<br>paragraph [0039] | |
| WO 2020/138067 A1 | 02 Jul. 2020 | (Family: none) | |

Form PCT/ISA/210 (patent family annex) (January 2015)

INTERNATIONAL SEARCH REPORT

International application No.
PCT/JP2021/016737

<Continuation of Box A>

A61K 31/4965(2006.01)i; A61K 31/07(2006.01)i; A61K 31/11(2006.01)i; A61K 31/121(2006.01)i; A61K 31/122(2006.01)i; A61K 31/137(2006.01)i; A61K 31/138(2006.01)i; A61K 31/216(2006.01)i; A61K 31/343(2006.01)i; A61K 31/351(2006.01)i; A61K 31/352(2006.01)i; A61K 31/357(2006.01)i; A61K 31/366(2006.01)i; A61K 31/395(2006.01)i; A61K 31/403(2006.01)i; A61K 31/4045(2006.01)i; A61K 31/4178(2006.01)i; A61K 31/4184(2006.01)i; A61K 31/4245(2006.01)i; A61K 31/426(2006.01)i; A61K 31/427(2006.01)i; A61K 31/436(2006.01)i; A61K 31/439(2006.01)i; A61K 31/4706(2006.01)i; A61K 31/506(2006.01)i; A61K 31/517(2006.01)i; A61K 31/519(2006.01)i; A61K 31/522(2006.01)i; A61K 31/5365(2006.01)i; A61K 31/5377(2006.01)i; A61K 31/56(2006.01)i; A61K 31/573(2006.01)i; A61K 31/58(2006.01)i; A61K 31/59(2006.01)i; A61K 31/661(2006.01)i; A61K 31/662(2006.01)i; A61K 31/7056(2006.01)i; A61K 31/706(2006.01)i; A61K 31/716(2006.01)i; A61K 36/04(2006.01)i; A61K 36/19(2006.01)i; A61K 38/06(2006.01)i; A61K 38/12(2006.01)i; A61K 38/17(2006.01)i; A61K 38/21(2006.01)i; A61K 45/00(2006.01)i; A61P 31/14(2006.01)i; A61P 43/00(2006.01)i

FI: A61K31/4965; A61P31/14; A61K45/00; A61P43/00 121; A61K31/137; A61K31/662; A61K31/4045; A61K31/436; A61K31/426; A61K31/343; A61K38/21; A61K31/522; A61K31/7056; A61K31/5365; A61K38/06; A61K36/19; A61K31/352; A61K31/706; A61K31/661; A61K31/427; A61K31/395; A61K31/4184; A61K31/4178; A61K31/4245; A61K31/122; A61K31/4706; A61K31/439; A61K38/17; A61K31/519; A61K31/403; A61K31/517; A61K31/506; A61K31/5377; A61K31/357; A61K31/366; A61K31/58; A61K31/573; A61K31/216; A61K31/121; A61K31/59; A61K31/716; A61K31/07; A61K31/11; A61K31/138; A61K31/56; A61K38/12; A61K31/351; A61K36/04

<Continuation of Box B>

A61K45/00; A61P31/14; A61P43/00; A61K36/04; A61K36/19; A61K38/06; A61K38/12; A61K38/17; A61K38/21; A61K31/07; A61K31/11; A61K31/121; A61K31/122; A61K31/137; A61K31/138; A61K31/216; A61K31/343; A61K31/351; A61K31/352; A61K31/357; A61K31/366; A61K31/395; A61K31/403; A61K31/4045; A61K31/4178; A61K31/4184; A61K31/4245; A61K31/426; A61K31/427; A61K31/436; A61K31/439; A61K31/4706; A61K31/4965; A61K31/506; A61K31/517; A61K31/519; A61K31/522; A61K31/5365; A61K31/5377; A61K31/56; A61K31/573; A61K31/58; A61K31/59; A61K31/661; A61K31/662; A61K31/7056; A61K31/706; A61K31/716

Form PCT/ISA/210 (extra sheet) (January 2015)

INTERNATIONAL SEARCH REPORT

International application No.
PCT/JP2021/016737

<Continuation of Box No. III>

Document 1: CAI, Q. et al., "Experimental Treatment with Favipiravir for COVID-19: An Open-Label Control Study.", Engineering, March 2020, 6, pp. 1192-1198

The claims are classified into the following 18 inventions.

(Invention 1) Invention containing (1) in claim 1, and claim 2

Document 1 indicates that: ribavirin, interferon, favipiravir, and lopinavir/ritonavir were applied to patients with SARS or MERS (page 1193, left column, third paragraph); and interferon α was inhaled in addition to favipiravir in order to confirm the effect of these medicines on COVID-19, and as a result, recovery in chest CT was more remarkable compared to the case where interferon α was inhaled in addition to lopinavir/ritonavir, and thus favipiravir is effective in SARS-Cov-2 infection (abstract, page 1193, right column, second paragraph, and table 2).

Document 1 is considered to disclose a pharmaceutical composition which is for treating coronavirus infectious diseases and contains interferon α and favipiravir, that is, a compound represented by general formula [1].

A pharmaceutical composition containing "(1) interferon" that is the first option, in the invention in claim 1 of the present application, cannot be said to provide a novel use in light of the disclosure of document 1, and lacks novelty, and thus does not have a special technical feature.

Therefore, parts containing "(1) interferon" in the invention in claim 1 for which the presence or absence of a special technical feature was determined are classified as invention 1.

In addition, claim 2 is classified as invention 1.

(Invention 2) Invention containing (2) in claim 1, and claim 3

In the invention containing "(2) a nucleic acid analogue" in claim 1, a nucleic acid analogue and interferon are not considered to contain a common component, but are considered to share the common technical feature of a pharmaceutical composition for treating coronavirus infectious diseases together with the compound represented by general formula [1]. However, as described above, said feature does not make a contribution over the prior art in light of the disclosure of document 1, and thus cannot be said to be a special technical feature. Moreover, there do not exist other identical or corresponding special technical features. Furthermore, the invention containing (2) in claim 1 is not substantially identical or equivalent to any of the claims classified as invention 1.

Therefore, the invention containing (2) in claim 1 cannot be classified as invention 1, but is classified as invention 2.

In addition, claim 3 is classified as invention 2.

Similarly, the other components are also classified as follows.

(Invention 3) Invention containing (3) in claim 1, and claim 4
(Invention 4) Invention containing (4) in claim 1, and claim 5
(Invention 5) Invention containing (5) in claim 1, and claims 6 and 20
(Invention 6) Invention containing (6) in claim 1, and claim 7
(Invention 7) Invention containing (7) in claim 1, and claim 8
(Invention 8) Invention containing (8) in claim 1, and claim 9
(Invention 9) Invention containing (9) in claim 1, and claim 10
(Invention 10) Invention containing (10) in claim 1, and claim 11
(Invention 11) Invention containing (11) in claim 1, and claim 12

Form PCT/ISA/210 (extra sheet) (January 2015)

**INTERNATIONAL SEARCH REPORT**

International application No.
PCT/JP2021/016737

(Invention 12) Invention containing (12) in claim 1, and claim 13
(Invention 13) Invention containing (13) in claim 1, and claim 14
(Invention 14) Invention containing (14) in claim 1, and claim 15
(Invention 15) Invention containing (15) in claim 1, and claim 16
(Invention 16) Invention containing (16) in claim 1, and claim 17
(Invention 17) Invention containing (17) in claim 1, and claim 18
(Invention 18) Invention containing (18) in claim 1, and claim 19

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0010569 A


### Non-patent literature cited in the description


- **YUTAKA HOSODA.** Senryou Kagaku. Gihodo Co., Ltd, 1957, 621
- **WANG C ; HORBY PW ; HAYDEN FG ; GAO GF.** A novel coronavirus outbreak of global health concern. *Lancet,* 2020, vol. 395, 470-3
- The species Severe acute respiratory syndrome-related coronavirus: classifying 2019-nCoV and naming it SARS-CoV-2. *Nat Microbiol.,* 02 March 2020
- *Coronavirus disease 2019 (COVID-19) situation reports,* 22 March 2020, vol. 62, https://www.who.int/docs/default-source/coronaviruse/situation-reports/20200322-sitrep-62-covid-19.pdf?sfvrsn=f7764c46 _2
- *ACS Central Science,* 2020, vol. 6 (3), 315-331
- **W. GREENE et al.** Protective Groups in Organic Synthesis. John Wiley & Sons, Inc, 2014, 895-1193
- **WANG M ; CAO R ; ZHANG L ; YANG X ; LIU J ; XU M et al.** Remdesivir and chloroquine effectively inhibit the recently emerged novel coronavirus [2019-nCoV] in vitro. *Cell Res.,* 2020, vol. 30, 269-71